(19) Europäisches Patentamt
European Patent Office
Office européen des brevets

(11) **EP 2 590 690 B1**

(12) **EUROPÄISCHE PATENTSCHRIFT**

(45) Veröffentlichungstag und Bekanntmachung des Hinweises auf die Patenterteilung:
**18.04.2018 Patentblatt 2018/16**

(21) Anmeldenummer: **11722315.6**

(22) Anmeldetag: **16.05.2011**

(51) Int Cl.:
*A61L 15/46* (2006.01)   *A61L 24/00* (2006.01)
*A61L 27/54* (2006.01)   *A61L 28/00* (2006.01)
*A61L 29/16* (2006.01)   *A61L 31/16* (2006.01)

(86) Internationale Anmeldenummer:
**PCT/EP2011/002405**

(87) Internationale Veröffentlichungsnummer:
**WO 2012/003899 (12.01.2012 Gazette 2012/02)**

(54) **ANTIBAKTERIELLES MEDIZINPRODUKT UND METHODE ZU DEREN HERSTELLUNG**

ANTIBACTERIAL MEDICINAL PRODUCT AND METHOD FOR PRODUCING SAME

DISPOSITIF MÉDICAL ANTIBACTÉRIEN ET PROCÉDÉ DE PRODUCTION DUDIT DISPOSITIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.07.2010 US 362888 P**

(43) Veröffentlichungstag der Anmeldung:
**15.05.2013 Patentblatt 2013/20**

(73) Patentinhaber: **Oerlikon Surface Solutions AG, Pfäffikon**
**8808 Pfäffikon (CH)**

(72) Erfinder:
• **COLMENARES MORA, Carmen Leonor**
**9479 Malans (CH)**
• **MUELLER, Arnd**
**7208 Malans (CH)**
• **JANSSEN, Albert Peter Gerhard**
**7000 Chur (CH)**

(74) Vertreter: **Kempkens, Anke et al**
**Hofgraben 486**
**86899 Landsberg (DE)**

(56) Entgegenhaltungen:
WO-A1-02/17984        WO-A1-2005/065603
WO-A2-2009/095705     DE-A1-102005 044 361
DE-A1-102008 001 014

## Beschreibung

Die vorliegende Erfindung betrifft ein Medizinprodukt insbesondere ein medizinisches Werkzeug oder Instrument mit auf einem Grundkörper aufgebrachter antibakterieller Hartstoffbeschichtung.

## Definitionen und Messverfahren

Im Sinne der Richtlinie 93/42/EWG des Rates vom 14. Juni 1993 über Medizinprodukte gelten als Medizinprodukte alle einzeln oder miteinander verbunden verwendeten Instrumente, Apparate, Vorrichtungen, Software, Stoffe oder anderen Gegenstände, einschließlich der vom Hersteller speziell zur Anwendung für diagnostische und/oder therapeutische Zwecke bestimmten und für ein einwandfreies Funktionieren des Medizinprodukts eingesetzten Software, die vom Hersteller zur Anwendung für Menschen für folgende Zwecke bestimmt sind:

- Erkennung, Verhütung, Überwachung, Behandlung oder Linderung von Krankheiten;

- Erkennung, Überwachung, Behandlung, Linderung oder Kompensierung von Verletzungen oder Behinderungen;

- Untersuchung, Ersatz oder Veränderung des anatomischen Aufbaus oder eines physiologischen Vorgangs;

- Empfängnisregelung,

und deren bestimmungsgemäße Hauptwirkung im oder am menschlichen Körper weder durch pharmakologische oder immunologische Mittel noch metabolisch erreicht wird, deren Wirkungsweise aber durch solche Mittel unterstützt werden kann.
Darüber hinaus im Sinne der Richtlinie 93/42/EWG des Rates vom 14. Juni 1993 über Medizinprodukte gilt als Zubehör ein Gegenstand, der selbst kein Produkt ist, sondern nach seiner vom Hersteller speziell festgelegten Zweckbestimmung zusammen mit einem Produkt zu verwenden ist, damit dieses entsprechend der vom Hersteller des Produkts festgelegten Zweckbestimmung des Produkts angewendet werden kann.
Im Sinne dieses Patents gilt als **Medizinprodukt** sowohl ein Medizinprodukt als auch ein Zubehör zu einem Medizinprodukt nach der Definitionen von Medizinprodukt und Zubehör gemäß der Richtlinie 93/42/EWG des Rates vom 14. Juni 1993 über Medizinprodukte aber auch zur Anwendung für Tiere und mit Ausnahme von:

- Endoprothesen beziehungsweise von Medizinprodukten, die im Körper eingepflanzt werden und permanent oder zumindest für einen längeren Zeitraum dort verbleiben sollen.

- Stoffen und weiteren Medizinprodukten oder Zubehören zu einem Medizinprodukt, die keine feste Oberfläche aufweisen.

Hartstoffe weisen eine große Härte und Verschleißfestigkeit auf. Diese Werkstoffe sind von großer technischer Bedeutung. Aufgrund der vielfältigen Eigenschaften und unterschiedlicher Anwendungsgebiete ist es schwierig, eine umfassende Definition für Hartstoffe zu finden. Folgende charakteristische Eigenschaften gelten für die meisten Hartstoffe: hohe Härte, hohe Verschleißfestigkeit, hohe Schmelzpunkte, chemische Beständigkeit, weitere Mechanische Eigenschaften, wie z. B. Zugfestigkeit, Dehnung oder Biegebruchfestigkeit hängen stark von der Art des Hartstoffes ab. Dies gilt ebenfalls für die elektrische Leitfähigkeit. Allgemein hängen die Eigenschaften der Hartstoffe eng mit der elektronischen Struktur dieser Verbindungen zusammen. Je nachdem ob metallische oder kovalente Bindungen vorliegen, ergeben sich deutliche Unterschiede zwischen metallischen und nichtmetallischen Hartstoffen.
Im Sinne der vorliegenden Erfindung wird für die Bezeichnung einer Beschichtung als **Hartstoffbeschichtung** speziell die Schichthärte oder Härte des Schichtsystems berücksichtigt und als Merkmal eine Härte von mindestens 1500 HV (Vickershärte) oder 17 GPa (Martenshärte).
Berichtete Härtewerte wurden nach dem Martens-Härteprüfverfahren mit einem "Fischerscope H100C" gemessen. Als Eindringkörper wurde eine Vickersdiamant angewendet. Das Martens-Härteprüfverfahren Verfahren ist in der DIN EN ISO 14577 (Metallische Werkstoffe - Instrumentierte Eindringprüfung zur Bestimmung der Härte und anderer Werkstoffparameter) genormt. Bei diesem Verfahren wird während der Belastungs- und Entlastungsphase kontinuierlich die Kraft und die Eindringtiefe gemessen. Die Martenshärte wird definiert als das Verhältnis der Maximalkraft zu der dazugehörigen Kontaktfläche und in der Einheit Newton pro Quadratmillimeter angegeben. Die Martenshärte $HM$ wird aus den Messwerten der Kraft/Eindringtiefe-Kurve während der Prüfkraftzunahme, bevorzugt nach dem Erreichen einer festgelegten Prüfkraft, berechnet und in diesem Fall bei Anwendung eines Vickersdiamants als Eindringkörper gilt die folgende Definition:

$$HM\left[Nmm^{-2}\right] = \frac{F}{A_s(h)} = \frac{F}{26.44 \cdot h^2}$$

wobei F... Prüfkraft, [n] und h... Eindringtiefe unter der Prüfkraft [mm]
Die gegebene Härtedefinition ist prinzipiell auch auf dünne Schichten anwendbar. Es sind jedoch einige Besonderheiten zu beachten. Beim Eindringen des Prüfkörpers erfolgt in der Regel gleichzeitig eine Verformung von Schicht und Substrat. Es wird also eine Verbundhärte $H_c$ ermittelt, für die folgende Beziehung gilt:

$$H_c = \frac{V_f}{V_f + V_s} \cdot H_f + \frac{V_s}{V_f + V_s} \cdot H_s$$

*wobei $H_f$, $H_s$... Schicht-, Substrathärte und $V_f$, $E_s$... deformiertes Schicht-, Substratvolumen*

Das deformierte Substratvolumen wird um so geringer, je kleiner die Eindringtiefe des Indenters und daher je kleiner die Eindringtiefe und damit die Prüfkraft, um so mehr nähert sich die ermittelet Verbundhärte der tatsächlichen Schichthärte. Bei Messungen der Härte von Schichtsystemen wird im allgemeinen (gemäß der Bückle-Regel) davon ausgegangen, dass bei Eindringtiefen

$$h \le \frac{1}{10} df \dots \frac{1}{7} df$$

*wobei df... Schichtdicke*

die ermittelte Härte annähernd gleich der Schichthärte. Im Rahmen der vorliegenden Erfindung wurden die berichteten Schichthärtewerte unter Berücksichtigung der Bückle-Regel ermittelt.

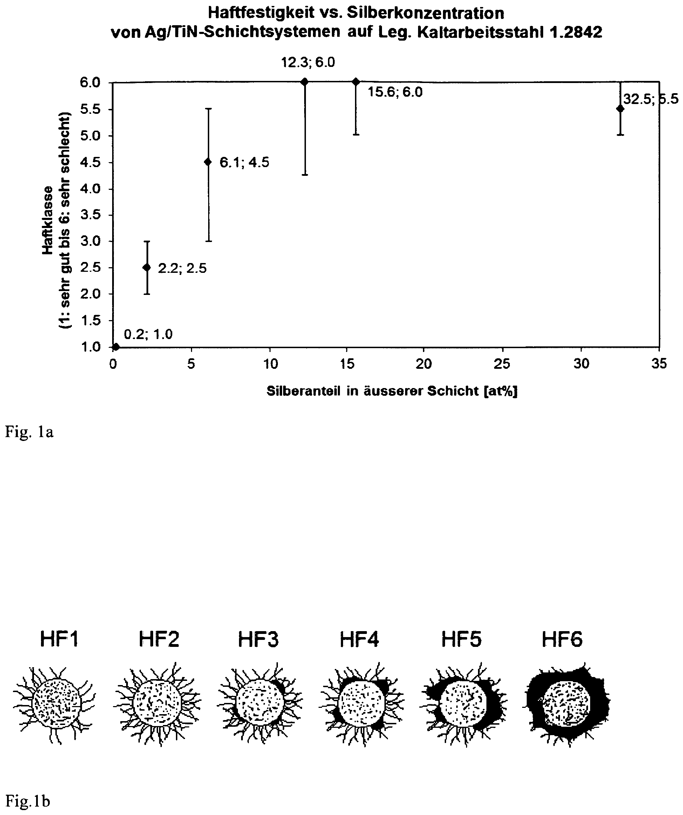

Die **Haftklasse** wurde nach Rockwelltest ermittelt. Bei der Überprüfung der Schichthaftung mittels Rockwelltest nach VDI-Richtlinie 3198 sowie 3824-4 wird die Größe und Art des Rissnetzwerkes sowie der Abplatzungen der Beschichtung im Randbereich eines HRC-Eindruckes beurteilt und daraus direkte Schlussfolgerungen zur Haftfestigkeit gemäß der in Fig.1 gezeigte Skizze bzw. Bilderreihe zur Bestimmung der Haftfestigkeitsklasse von PVD-Schichten auf Werkzeug oder Schnellarbeitsstahl (HF1: sehr gut bis HF6: sehr schlecht). Zur Verwendung dieser Methode müssen Rockwellhärte vom Grundwerkstoff und Schichtdicke oder Dicke des Schichtsystems jeweils nicht kleiner 54 HRC und nicht größer 5 $\mu$m sein. Die Auswertung wird mittels HRC-Eindruck (150kp) bei 100facher Vergrößerung direkt am Mikroskop gemacht.

Als Messgerät für die Farbmessung der Oberfläche der Schichtsysteme wurde ein 3-Bereichs-Farbmeßgerät MIKRO COLOR II der Fa. Dr. Lange verwendet, das mit einer Xenon-Blitzlampe auf der Probenoberfläche diffuse Reflexionen erzeugt und diese anschließend gemäß DIN 5033 misst. Als Bezugsstandard für die Kalibrierung wurde ein Weißstandard mit Normfarbwerte X: 84.6 Y: 89.4 und Z: 91.8 verwendet. Nach jeder Farbmessung wurden die daraus resultierenden Farbwerte im Display des Farbmessgerät entsprechend dem CIELAB dreidimensionalen Koordinatensystem (L*a*b*) angezeigt, wobei die a*-Achse den Grün- oder Rotanteil einer Farbe mit Werten von -150 bis 100 beschreibt (negative Werte für Grün und positive Werte für Rot), die b*-Achse den Blau- oder Gelbanteil einer Farbe mit Werten von -100 bis 150 beschreibt (negative Werte für Blau und positive Werte für Gelb) und die L*-Achse die Helligkeit (Luminanz) der Farbe mit Werten von 0 bis 100 beschreibt.

## Stand der Technik

Heutzutage sind verschiedene Produkte in Markt vorhanden, die beanspruchen, antibakterielle oder antimikrobielle Eigenschaften zu haben. Besonders zur Vorbeugung von Infektionskrankheiten wird versucht, Hygiene- und Medizinprodukte mit antibakteriellen bzw. antimikrobiellen Eigenschaften auszustatten. Bekannt ist die Verwendung von Silber und dessen Ionen als anorganisches Biozid zur Behandlung von Materialien, die danach im Laufe der Zeit das in ihnen enthaltene Biozid freisetzen, somit die Ansiedlung oder Vermehrung von Mikroorganismen auf diesen Materialien und sogar in ihrer Umgebung verringert oder vollständig verhindert wird. Zu diesem Zweck sind bisher mehrere antimikrobielle Silber-Technologien erforscht und entwickelt worden. Andere bekannte anorganische Biozide sind auch zum Beispiel Kupfer und Zink, die aus bestimmten Gründen wie zum Beispiel höhere Toxizität und weniger effiziente biozidische Wirksamkeit im Vergleich mit Silber für medizintechnische Anwendungen weniger gefragt sind.

Durch die HyProtect™ - Technologie werden zum Beispiel dünne (< 200 nm) antimikrobielle Beschichtungen auf, unter anderem, Medizinprodukte appliziert, die aus einer Kombinationsschicht aus reinem Silber, das über einen PVD (physical vapor deposition) Prozess abgeschieden wird und einer Siliziumoxidschicht, die über einen CVD (chemical vapor deposition) Prozess abgeschieden wird, bestehen. Diese Technologie gestattet die Erzeugung von vor allem gewünschten optischen und antimikrobiellen Eigenschaften auf verschiedenen Oberflächen, aber ermöglicht keine richtige Verbesserung von Verschleißschutz.

Bekannt ist auch die Verwendung von physikalischen und/oder chemischen und/oder plasmaunterstützten chemischen Beschichtungsmethoden unter Vakuum, d.h. PVD- und/oder CVD- und/oder PACVD (plasma assisted CVD) Beschichtung von Medizinprodukten mit Hartstoffschichten (wie zum Beispiel mit TiN, CrN und DLC). Dadurch kann man in der Regel gewünschte optische sowie tribologische Eigenschaften auf der Oberfläche eines Medizinprodukts erreichen. Allerdings zeigen diese keine ausreichende antimikrobielle Wirksamkeit.

Die Anmelderin hat in ihrem Produktportfolio verschiedene Beschichtungen, die speziell für Medizinprodukte entwickelt worden sind, um auf die Oberfläche von diesen Medizinprodukten neben bestimmte tribologische Eigenschaften und auch definierte optische Eigenschaften zu übertragen. Insbesondere werden durch diese Beschichtungen neben erhöhter Verschleißbeständigkeit und verbesserter Reibeigenschaften auch definierte glänzende oder glanzlose Farben erzielt.

Beispiele von Schichtsysteme und Farben sind:

- TiN → Gold,

- AlTiN oder a-C:H → Schwarz,

- TiAlN → Bronze,

- CrN → Silber und

- WC/C → Grau.

Beispiele von Schichtsystemen und tribologische und/oder gewünschte optische Eigenschaften für Medizinprodukte sind:

- TiN → zur sicheren Kennzeichnung von Implantate-Probierschäften

- TiN und WC/C → zum Verschleißschutz und zur Reibminderung von Bohrbuchsenkörpern

- WC/C und CrN → zur Verschleißschutz von Dentalbohrern

- WC/C → zur Reibminderung und/oder zum Verschleißschutz von Knochenverschraubungswerkzeugen, Zahnimplantatsverschraubungswerkzeugen, Knochenbohrern, Knochensägen, Implantationspistolen, Arterienreinigungsfedern

- WC/C → zur Übertragung von Antireflexeigenschaften auf Augenchirurgiemesser

- DLC → zum Korrosionsschutz und/oder zur sicheren Kennzeichnung von Zahnsteinentfernern und/oder auch Übertragung von Antireflexeigenschaften. (DLC wird häufig auch a:CH genannt)

Mehrere Forschungsarbeiten über die PVD- u/o CVD- u/o PACVD-Abscheidung von antimikrobiellen Ag-haltigen Hartstoffschichten (wie zum Beispiel von Ag-TiN, Ag-CrN und Ag-a-C:H) zur medizintechnischen Anwendung, vor allem für die Beschichtung von Endoprothesen wurden auch bereits von verschiedenen Forschungsinstitutionen veröffentlicht. Bei diesen Arbeiten wurde versucht, antimikrobielle und/oder tribologische Eigenschaften sowie in manchen Fällen auch Biokompatibilität der so aufgebauten Schichtsysteme zu erforschen, aber ganz ohne Berücksichtigung von optischen Eigenschaften wie Farbe und Reflexion, die insbesondere für manche medizintechnische Werkzeuge und Instrumenten eine sehr wichtige Rolle spielen.

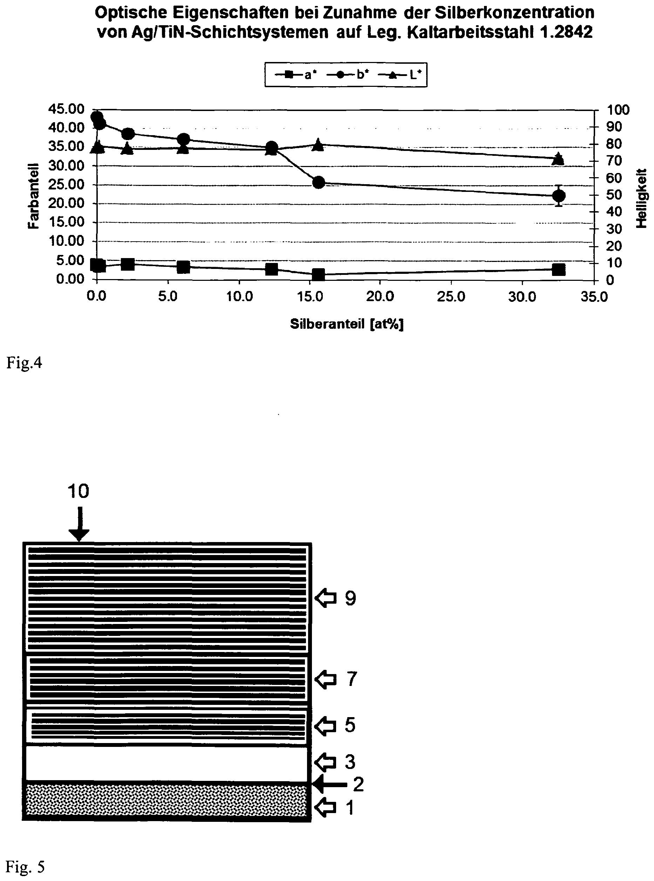

Bei eigenen Entwicklungsarbeiten könnte festgestellt werden, dass durch Silber-Dotierung von Hartstoffschichten am Beispiel vom Titaniumnitride (TiN), entscheidende Schichtparameter wie Haftfestigkeit, Härte und Rauhigkeit negativ beeinflusst werden können (siehe Fig.1, 2, 3). Insbesondere konnte festgestellt werden dass je höher der Silberanteil war desto niedriger die Haftfestigkeit zum Grundkörper. Darüber hinaus konnten auch durch die Dotierung mit Silber die optischen Eigenschaften des ursprünglichen Schichtsystems beeinflusst werden (siehe Fig.4).

In der Offenlegungsschrift DE102008001014A1 wird ein Schichtaufbau vorgeschlagen, durch die die Herstellung eines antimikrobiellen und nicht zytotoxischen Schichtmaterials ermöglicht werden soll. Das offenbarte Schichtmaterial umfassend eine Trägerschicht, zumindest eine auf der Trägerschicht aufgebrachte Biozid-Schicht mit einem bioziden Wirkstoff, und eine auf die Biozid-Schicht aufgebrachte Transportkontrollschicht mit einer Gasdurchlässigkeit für Sauerstoff ($O_2$) von 50 bis unter 100 ($cm^3$ bär)/Tag $m^2$).

Durch die Gasdurchlässigkeit für Sauerstoff der Transportkontrollschicht kann gemäß DE102008001014A1 ermöglicht werden, dass der biozide Wirkstoff aus der Biozid-Schicht durch die Transportkontrollschicht hindurch in einer antimikrobiell wirksamen und gleichzeitig nicht zytotoxischen Menge abgegeben wird.

In DE102008001014A1 wird es erwähnt, dass vorzugsweise die anorganische Biozide Silber, Kupfer und Zink oder Mischungen dieser drei Materialien als biozide Wirkstoffe in der Biozid-Schicht verwendet werden können. Jedoch gibt DE102008001014A1 keinen Hinweis für die Herstellung einer Hartstoffschicht mit antimikrobiellen Eigenschaften und berichtet auch nicht über mögliche Einflüsse der Konzentration des Biozids auf gewisse Eigenschaften der Biozid-haltigen Schicht oder des gesamten Schichtmaterials, wie beispielweise Haftfestigkeit, Härte und Rauhigkeit.

WO2009/095705A2 beschreibt metallische biomedizinische Artikel mit einer mittels Elektronenstahl-PVD abgeschiedenen Beschichtung, die vorzugsweise zwei Silber-Metall-Nitridschichten umfasst., wobei die Silberkonzentration in der äusseren Schicht höher als in der inneren Schicht ist und die Silberkonzentration 0.1 bis 99 at%, vorzugsweise 0.5 bis 35 at % oder 1 bis 25 at% beträgt. Jedoch berichtet WO2009/095705A nicht über mögliche Einflüsse der Silberkonzentration auf gewisse Eigenschaften der Biozid-haltigen Schicht oder des gesamten Schichtmaterials, wie beispielweise Haftfestigkeit und optische Eigenschaften.

**Aufgabe der Erfindung**

Aufgabe der vorliegenden Erfindung ist es, eine für Medizinprodukte geeignete Beschichtung anzugeben, welche zu vorbestimmten antibakteriellen Eigenschaften und zu einer Verbesserung der bereits vom Substrat aufgewiesenen tribologische Eigenschaften wie zum Beispiel Verschleißbeständigkeit und Reibminderung führt. Dafür muss die Beschichtung hauptsächlich eine ausreichende Haftung zum Grundkörper des Medizinproduktes aufweisen können. Die Korrosionsbeständigkeit soll erhalten bleiben oder zumindest lediglich im zulässigen Rahmen abnehmen. Dabei wird vorzugsweise versucht, den Medizinprodukten diejenigen optischen Eigenschaften zu geben, die zu einer Akzeptanz der Anwender führen. Insbesondere ist es Aufgabe der vorliegenden Erfindung solche Eigenschaften auf Oberflächen von genannten Medizinprodukten, insbesondere von Werkzeugen und Instrumenten mittels Beschichtung, insbesondere mittels PVD und/oder PACVD Beschichtungen zu erzielen.

Darüber hinaus muss berücksichtigt werden, dass die Substrattemperatur beim Beschichtungsprozess auf die Temperaturbeständigkeit des Substratmaterials geachtet werden muss, d.h. die maximal zugelassene Temperatur hinsichtlich des Substratmaterials darf nicht überschritten werden.

Insbesondere sollte in der Regel bei der Beschichtung der Medizinprodukte wie zum Beispiel entsprechende Instrumente und Werkzeuge aufgrund der üblich verwendeten Substratsorten, wie zum Beispiel Edelstahl 1.4542 eine Temperatur von 300 °C nicht überschritten werden.

**Lösung der Aufgabe**

Die Aufgabe wird erfindungsgemäß durch eine auf einem Grundkörper (1) eines Medizinproduktes aufgebrachte antibakterielle Hartstoffbeschichtung mit Biozid umfassend gelöst. Die Hartstoffbeschichtung enthält mindestens eine innere Schicht (5) mit einer Dicke d1 von mindestens 0.2 $\mu$m und eine äußere Schicht (9) mit einer Dicke d2 von mindestens 0.5 $\mu$m. Die innere Schicht (5) ist zwischen der äußeren Schicht und dem Grundkörper (1) angeordnet und die äußere Schicht (9) enthält eine über deren gesamte Schichtdicke im Wesentlichen konstante Biozidkonzentration bcI größer oder gleich 2 at%. Angaben zur Biozidkonzentration beziehen sich im Rahmen dieser Beschreibung immer auf eine Mittelung über mindestens 20 nm Tiefe. Die innere Schicht (5) weist eine Biozidkonzentration mit Maximalwert bcII größer oder gleich 0.2 at% auf. Die Biozidkonzentration der inneren Schicht (5) ist über deren gesamten Schichtdicke kleiner als die im Wesentlichen konstante Biozidkonzentration bcI der äußeren Schicht (9). Die Silberkonzentration in der inneren Schicht (5) entlang ihrer Schichtdicke kann variieren und insbesondere in Richtung vom Gründkörper (1) zu äußerer Schicht (9) zunehmen, wobei zwischen der inneren Schicht (5) und der äußeren Schicht (9) mindestens eine Zwischenschicht (7) mit Biozidkonzentration bcIII angeordnet ist, wobei bcIII größer bcI ist. Damit verfügt das erfindungsgemäß gestaltete Schichtsystem über eine verbesserte Haftung zum Substrat im Vergleich zu einem Schichtsystem ohne erfindungsgemäße innere Schicht.

**Beschreibung der Erfindung**

Die Erfindung wird anschließend anhand von Beispielen erläutert, wobei auf die gezeigten Figuren Bezug genommen wird:
Die Figuren zeigen:

Fig. 1a: Verhalten: Haftfestigkeit vs. Silberkonzentration von Ag/TiN-Schichtsystemen ohne erfindungsgemäße eingebaute innere Schicht abgeschieden auf polierten (Ra: 0.05 $\mu$m) Leg. Kaltarbeitsstahl 1.2842.

Fig. 1b Schematische Darstellung der Haftfestigkeitsklassen

Fig.2: Verhalten: Martenshärte vs. Silberkonzentration von Ag/TiN-Schichtsystemen abgeschieden auf polierten (Ra: 0.05 $\mu$m) Leg. Kaltarbeitsstahl 1.2842.

Fig.3: Verhalten: Rauheit vs. Silberkonzentration von Ag/TiN-Schichtsystemen abgeschieden auf polierten (Ra: 0.05 $\mu$m) Leg. Kaltarbeitsstahl 1.2842.

Fig.4: Verhalten: Optische Eigenschaften am Beispiel von Farbwerten nach dem CIELAB-System vs. Silberkonzentration von Ag/TiN-Schichtsystemen abgeschieden auf polierten (Ra: 0.05 $\mu$m) Leg. Kaltarbeitsstahl 1.2842.

Fig.5 : Beispielhaftes Design einer Beschichtung bzw. eines Schichtsystems für die erfindungsgemäße Herstellung von antibakteriellen Medizinprodukten, wobei:

(1)... Grundkörper

(2)... mechanische und/oder elektrochemische Vorbehandlung zur Erhöhung der Haftfestigkeit

(3)... Haftschicht und/oder Verschleißschutzschicht

(5)... erfindungsgemäß innere biozidumfassende Schicht

(7)... biozidumfassende Zwischenschicht mit höherer Biozidkonzentration als die von der äußere Schicht

(9)... äußere Schicht

(10)... mechanische Nachbehandlung wie z.B. Polieren zur Reduzierung der Rauheit

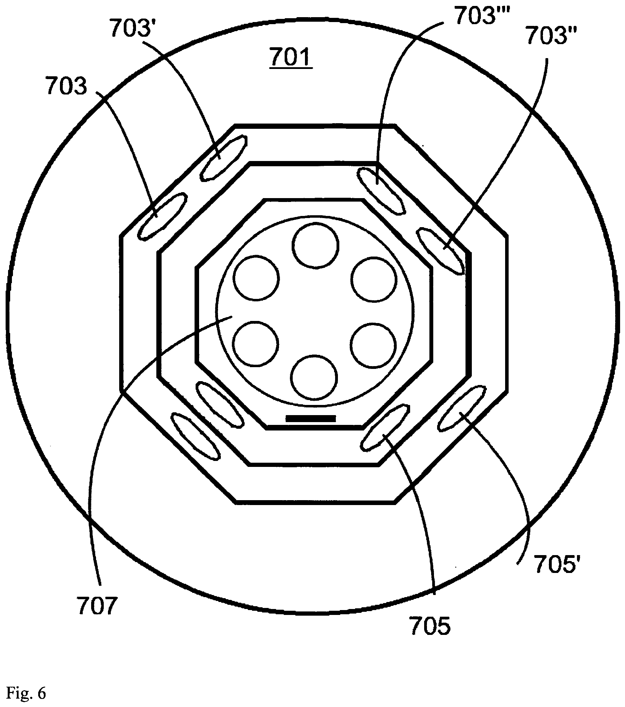

Fig.6: Beispielhafte Anordnung einer Beschichtungsanlage zur erfindungsgemäßen Herstellung von antibakteriellen Medizinprodukten

Verschiedene PVD-Beschichtungsmethoden wurden für die Herstellung von silberdotierten TiN-Schichten verwendet.
**Beispiel 1:** Mittels eines kombinierten Arc/Sputter-Prozesses bzw. eines hybrid AIP+MSIP-Prozesses wurden silberdotierte TiN-Schichtsysteme

bzw. Ag/TiN-Schichtsysteme hergestellt. In Fig.6 ist schematisch eine Anordnung einer Vakuumbeschichtungsanlage 701 dargestellt, womit die biozid wirkende Schichtsysteme auf den Grundkörper von Medizinprodukten und/oder Prüfkörpern abgeschieden wurden. Als Materialquelle wurden getrennte Titantargets 703, 703' 703" 703'" und Silber-Targets 705, 705' angewendet. Schichtsysteme mit unterschiedlichen Silberkonzentrationen wurden durch die Variation von Arc-Strom an den Ti-Targets 703, 703' 703" 703'", Sputter-Leistung an den Ag-Targets 705, 705' und Biasspannung am Substrat hergestellt. Darüber hinaus konnte man die Silberkonzentration durch die Variation der Anzahl der aktiven Titan- und/oder Silber-Targets variieren. Die Ag/TiN-Schichten wurden druckgeregelt in Ar/$N_2$-Atmosphäre bei einem Prozessdruck von 0.02 mbar abgeschieden. Die Substrate befanden sich während der Beschichtung auf einer Karusellanordnung 707 mit Zweifach- und Dreifachrotation. Die Rotationsgeschwindigkeit der Substrate wurde konstant gehalten. Auf die Oberfläche von Prüfkörper oder Medizinprodukte aus verschiedenen Stahlsorten und auch aus Hartmetall wurde nach entsprechenden Heiz- und Ätzprozesse in der Vakuumbeschichtungskammer eine sehr dünne Haftschicht aus TiN (Dicke $\leq 0.3\ \mu m$) mittels AIP-Techniken abgeschieden und anschließend die Ag/TiN-Schicht bei konstanten Prozessparameter mittels eine Kombination von AIP- und MSIP-Techniken wie oben beschrieben abgeschieden. In den Figuren 1 bis 4 wurde den Einfluss der Silberkonzentration auf die Härte, Rauheit, Optische Eigenschaften dargestellt, die bei verschiedenen Ag-Sputterleistungen und ansonsten gleichen Prozessparametern abgeschieden wurden. Es konnte festgestellt werden (wie in Fig.1a dargestellt), dass bei erhöhten Silberkonzentrationen die Haftfestigkeit stark verschlechtert wird.

Die Erfinder haben festgestellt, dass durch den Aufbau einer inneren Schicht 5 zwischen dem Gründkörper 1 und der mit erhöhter Silberkonmzentration ausgestatteten äußeren Schicht 9 wie im Fig.5 dargestellt, die Haftfestigkeit von Ag/Ti-Schichtsysteme mit erhöhter Silbergehalt deutlich verbessert werden kann.

Dis gilt auch dann noch, wenn zwischen innerer Schicht 5 und äusserer Schicht 9 eine Zwischenschicht 7 mit gegenüber der äusseren Schicht 9 erhöhten Konzentration vorgesehen ist, die als Silberreservoir für die äussere Schicht 9 dienen kann.

Zusätzlich kann noch eine Haftschicht 3 oder Verschleisschutzschicht 3 zwischen Grundkörper 1 und innerer Schicht 5 vorgesehen sein, wodurch die Haftung noch weiter gesteigert wird.

Wie im Beispiel oben gezeigt konnte mittels der erfindungsgemässen Schichtfolge und dem Verfahren die Haftfestigkeit der Schichten bei erhöhtem Silbergehalt deutlich verbessert werden.

**Beispiel 2:** Eine weitere Möglichkeit gemäss folgendem Beispiel 2 besteht darin das Biozid in DLC-Schichten einzubauen. Ausgehend vom Substrat wird beispielsweise eine Haftschicht, zum Beispiel Chrom aufgebracht. Anschließend wird eine DLC Schicht durch PACVD abgeschieden und gleichzeitig das Silbertarget aktiviert. Auch hier wird zunächst wenig Silber zum Beispiel durch eine niedrige Sputterleistung eingebracht. Dann wird die Konzentration erhöht, beispielsweise durch Erhöhung der Sputterleistung bei ansonsten gleich bleibenden Beschichtungsparametern. Am Schluss wurde die Silberkonzentration während der Beschichtung konstant hoch gehalten um die äussere Schicht mit konstanter Biozidkonzentration herzustellen. Wiederum entstand eine erfindungsgemässe Schicht mit gegenüber dem Stand der Technik verbesserten Hafteigenschaften.

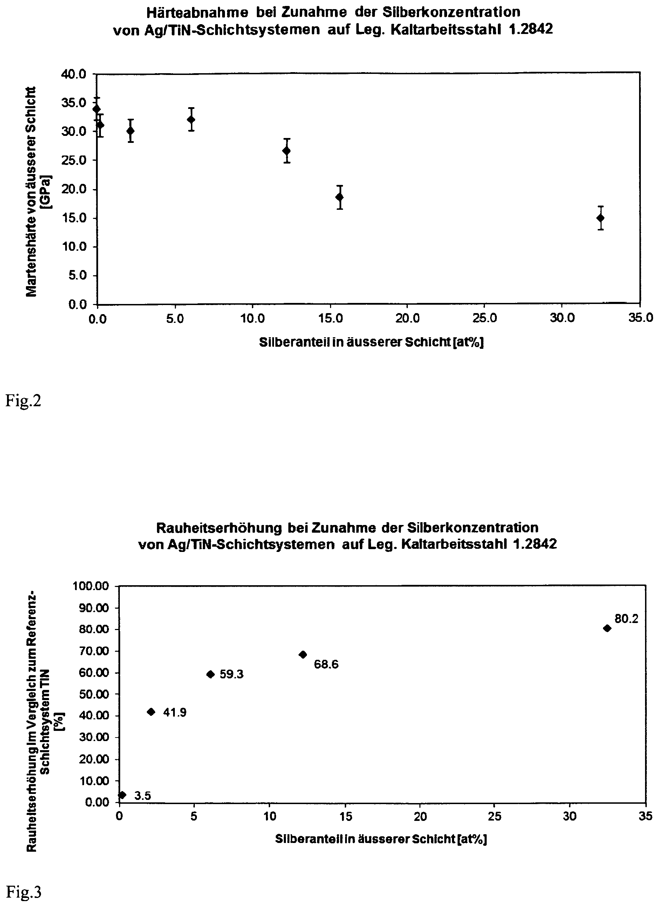

Es stellt sich nun die Frage, wodurch der Konzentrationsgehalt des Biozids zu beschränken ist. Eigentlich würde man vermuten, dass je höher die Biozidkonzentration ist, umso besser ist die Wirkung. Dem widersprechen allerdings erstaunlicherweise die von den Erfindern durchgeführten Messungen. Es kommt hinzu, dass ab einer Konzentration über 15at% die Härte der Schicht wie in Figur 2 dargestellt stark abnimmt und, wie in Figur 4 dargestellt, der Farbeindruck sich stark ändert. Es wird daher vorgeschlagen Biozidonzentrationen von bis zu 15at%, vorzugsweise von bis zu 13at% zu verwenden.

Es kann vorkommen, dass durch das Einbringen des Silbers die Oberfläche des beschichteten Produktes insbesondere Medizinproduktes eine erhöhte Rauheit aufweisst, wie zum Beispiel in der Figur 3 dargestellt. Die Erfinder haben festgestellt dass erhöhte Rauheiten zu einer Verminderung des biozidalen Effektes führt. Dies kann dadurch vermindert werden, dass die Oberfläche einer Nachbehandlung, insbesondere einer mechanischen Nachbehandlung wie Polieren, Nasstrahlen oder Läppen oder einer geeigneten chemischen Politur unterzogen wird. Aufgrund der Konstanz der Biozidkonzentration in der äusseren Schicht hat diese Nachbehandlung im Wesentlichen keinen Einfluss auf die Biozidale Wirkung bei der Anwendung des Medizinproduktes.

**Patentansprüche**

1. Medizinprodukt mit auf einem Grundkörper (1) aufgebrachter antibakterieller Hartstoffbeschichtung mit Biozid umfassend mindestens eine innere Schicht (5) mit einer Dicke d1 von mindestens 0.2 $\mu m$ und eine äußere Schicht (9) mit einer Dicke d2 von mindestens 0.5 $\mu m$, wobei die innere Schicht (5) zwischen der äußeren Schicht (9) und dem Grundkörper (1) angeordnet ist und die äußere Schicht (9) eine über deren gesamte Schichtdicke im Wesentlichen konstante über mindestens 20 nm Tiefe gemittelte Biozidkonzentration bcl größer oder gleich 2 Atomprozent enthält, **dadurch gekennzeichnet, dass** das Biozid, ein anorganisches Biozid oder eine Kombination von anorganischen Bioziden ist, wobei die innere Schicht (5) eine über 20 nm Tiefe gemittelte Biozidkonzentration mit Maximalwert bcII größer oder gleich 0.2 Atomprozent aufweist und die Biozidkonzentration der inneren Schicht (5) über deren gesamten Schichtdicke kleiner als die im Wesentlichen konstante Biozidkonzentration bcI der äußeren Schicht (9) ist, wobei zwischen der inneren Schicht (5) und der äußeren Schicht (9) mindestens eine Zwischenschicht (7) mit Biozidkonzentration bcIII angeordnet ist, wobei bcIII größer bcI ist.

2. Medizinprodukt nach Anspruch 1, **dadurch gekennzeichnet, dass** das anorganische Biozid Silver ist, oder die Kombination von anorganischen Bioziden Silver umfasst.

3. Medizinprodukt nach einem der vorangehenden Ansprüche, **dadurch gekennzeichnet, dass** zwischen der inneren Schicht (5) und dem Grundkörper (1) mindestens eine Haftschicht und/oder Verschleißschutzschicht und/oder Hartstoffschicht (3) ohne Biozid angeordnet ist.

4. Medizinprodukt nach einem der vorangehenden Ansprüche, **dadurch gekennzeichnet, dass** zwischen der inneren Schicht und dem Grundkörper oder gegebenenfalls zwischen der inneren Schicht und der Haftschicht mindestens eine Verschleißschutzschicht, die eine Härte größergleich 1500 HV aufweist und dadurch eine Stützwirkung für die daraufgelegten Schichten bietet.

5. Medizinprodukt nach einem der vorangehenden Ansprüche, **dadurch gekennzeichnet, dass** die höchste Biozidkonzentration nicht höher als 15 Atomprozent ist, vorzugsweise nicht höher als 13 Atomprozent.

6. Medizinprodukt nach einem der vorangehenden Ansprüche, **dadurch gekennzeichnet, dass** die Hartstoffbeschichtung bzw. das Schichtsystem TiN, TiAlN, AlTiN, CrN, WC/C und/oder a-C:H enthält.

7. Methode zur Herstellung eines Medizinprodukts nach einem der vorangehenden Ansprüche, **dadurch gekennzeichnet, dass** der angewendete Beschichtungsprozess zur Abscheidung des Schichtsystems ein PVD (physical vapor deposition) oder ein kombinierter PVD + PA-CVD (plasma assisted chemical vapor deposition) Prozess ist.

8. Methode nach Anspruch 7, **dadurch gekennzeichnet, dass** der angewendete PVD-Prozess ein AIP (arc ion plating) und/oder ein MSIP (magnetron sputter ion plating) und/oder ein Hybrid AIP+MSIP Prozess umfasst.

9. Methode nach Anspruch 8, **dadurch gekennzeich-** **net, dass** beim Beschichtungsprozess mindestens ein festes biozidhaltiges Target als Biozidquelle angewendet wird, das ein oder mehrere Biozide enhält.

10. Methode nach Anspruch 9, **dadurch gekennzeichnet, dass** die Methode einen Schritt umfasst, in dem mindestens ein biozidhaltiges Target mittels MSIP-Techniken und mindestens ein nicht biozidhaltiges Target mittels AIP-Techniken gleichzeitig in einem Edelgas/Reaktivgas Atmosphäre betrieben werden, wobei die Biozidkonzentration in den genannten Biozid umfassenden Schichten durch die Variation der Sputter-Leistung an dem mindestens einem biozidhaltigen Target und/oder durch die Variation der Arc-Strom an dem mindestens einem nicht biozidhaltigen Target gesteuert wird.

11. Methode nach einem der vorangehenden Ansprüche 7 bis 10 **dadurch gekennzeichnet, dass** die Oberfläche der Grundkörper (2) vor der Beschichtung mechanisch oder elektrochemisch behandelt wird.

12. Methode nach einem der vorangehenden Ansprüche 7 bis 11 **dadurch gekennzeichnet, dass** die beschichtete Oberfläche (10) nach der Beschichtung mechanisch behandelt insbesondere poliert wird.

## Claims

1. Medical product with an antibacterial hard material coating applied to a main body (1) and which comprises a biocide, including at least one inner layer (5) with a thickness d1 of at least 0.2 $\mu$m and one outer layer (9) with a thickness d2 of at least 0.5 $\mu$m, wherein the inner layer (5) is arranged between the outer layer (9) and the main body (1) and the outer layer (9) contains a biocide concentration bcI greater than or equal to 2 atomic percent, the concentration being essentially constant throughout its entire layer thickness and averaged over at least 20nm depth, **characterized in that** the biocide is a non-organic biocide or a combination of non-organic biocides, wherein the inner layer (5) has a biocide concentration averaged over 20 nm depth with a maximum value bcII greater than or equal to 0.2 atomic percent and the biocide concentration of the inner layer (5) over its entire layer thickness is smaller than the essentially constant biocide concentration bcI of the outer layer (9), wherein between the inner layer (5) and the outer layer (9) there is at least one intermediate layer (7) with a biocide concentration bcIII, wherein bcIII is greater than bcI.

2. Medical product according to claim 1, **characterized in that** the non-organic biocide is silver or the combination of non-organic biocides comprises silver.

3. Medical product according to one of the preceding claims, **characterized in that** at least one adhesive layer and/or anti-wear protection layer and/or hard material layer (3) without biocide is arranged between the inner layer (5) and the main body (1).

4. Medical product according to one of the preceding claims, **characterized in that** between the inner layer and the main body or, where appropriate, between the inner layer and the adhesive layer there is at least one anti-wear protection layer that has a hardness of equal to or greater than 1500 HV and thus provides a supporting effect for the overlaid layers.

5. Medical product according to one of the preceding claims, **characterized in that** the maximum biocide concentration is not greater than 15 atomic percent, preferably not greater than 13 atomic percent.

6. Medical product according to one of the preceding claims, **characterized in that** the hard material coating or layer system include TiN, TiAlN, AlTiN, CrN, WC/C and/or a-C:H.

7. Method for producing a medical product according to one of the preceding claims, **characterized in that** the applied coating process for depositing the layer system is a PVD (physical vapor deposition) or a combined PVD + PA-CVD (plasma assisted chemical vapor deposition) process.

8. Method according to claim 7, **characterized in that** the applied PVD process comprises an AIP (arc ion plating) and/or a MSIP (magnetron sputter ion plating) and/or a hybrid AIP+MSIP process.

9. Method according to claim 8, **characterized in that** during the coating process at least one solid target containing one or more biocides is used as a biocide source.

10. Method according to claim 9, **characterized in that** the method comprises a step where at least one target containing biocide is operated by means of MSIP techniques and, simultaneously, at least one target not containing biocide is operated by means of AIP techniques in a noble gas/reactive gas atmosphere, wherein the biocide concentration in said layers comprising biocide is controlled by varying the sputtering power of the at least one target containing biocide and/or by varying the arc current of the at least one target not containing biocide.

11. Method according to one of the preceding claims 7 to 10, **characterized in that** the surface of the main body (2) is subjected to mechanical or electrochemical treatment before the coating process.

12. Method according to one of the preceding claims 7 to 11, **characterized in that** the coated surface (10) is subjected to mechanical treatment, in particular polishing treatment, after the coating process.

**Revendications**

1. Produit médical ayant un revêtement de matériau dur antibactérien appliqué sur un corps de base (1) et comprenant un biocide, contenant au moins une couche intérieure (5) ayant une épaisseur d1 d'au moins 0,2 $\mu$m et une couche extérieure (9) ayant une épaisseur d2 d'au moins 0,5 $\mu$m, la couche intérieure (5) étant disposée entre la couche extérieure (9) et le corps de base (1) et la couche extérieure (9) contenant une concentration du biocide bcI supérieure ou égale à 2 pour cent atomique, la concentration étant essentiellement constante sur toute son épaisseur de couche et sa moyenne étant calculée sur au moins 20 nm de profondeur, **caractérisé par le fait que** le biocide est un biocide inorganique ou une combinaison de biocides inorganiques, la couche intérieure (5) ayant une concentration du biocide dont la valeur moyenne est calculée sur 20 nm de profondeur et qui a une valeur maximale bcII supérieure ou égale à 0.2 pour cent atomique et la concentration du biocide de la couche intérieure (5) sur toute son épaisseur de couche étant inférieure à la concentration du biocide bcI essentiellement constante de la couche extérieure (9), entre la couche intérieure (5) et la couche extérieure (9) il y ayant au moins une couche intermédiaire (7) avec une concentration du biocide bcIII, bcIII étant supérieure à bcI.

2. Produit médical selon la revendication 1, **caractérisé par le fait que** le biocide inorganique est de l'argent ou la combinaison de biocides inorganiques comprend de l'argent.

3. Produit médical selon l'une des revendications précédentes, **caractérisé par le fait que,** au moins une couche adhésive et/ou une couche de protection contre l'usure et/ou une couche de matériau dur (3) sans biocide est disposée entre la couche intérieure (5) et le corps de base (1).

4. Produit médical selon l'une des revendications précédentes, **caractérisé par le fait que**, entre la couche intérieure (5) et le corps de base (1), ou, le cas échéant, entre la couche intérieure (5) et la couche adhésive il y a au moins une couche de protection contre l'usure qui a une durêté supérieure ou égale à 1500 HV et qui offre ainsi un effet d'appui aux couches mises au-dessus.

**5.** Produit médical selon l'une des revendications précédentes, **caractérisé par le fait que** la concentration maximale de biocides n'est pas supérieure à 15 pour-cent atomique, de préférence non supérieure à 13 pour-cent atomique.

**6.** Produit médical selon l'une des revendications précédentes, **caractérisé par le fait que** le revêtement de matériau dur ou le système de couches contient TiN, TiAlN, AlTiN, CrN, WC/C et/ou a-C:H.

**7.** Méthode de fabrication d'un produit médical selon l'une des revendications précédentes, **caractérisé par le fait que** le processus de revêtement appliqué pour déposer le système de couches est un processus de PVD (physical vapor déposition) ou un processus combiné de PVD et PA-CVD (plasma assisted chemical vapor déposition).

**8.** Méthode selon la revendication 7, **caractérisé par le fait que** le processus de PVD appliqué comprend un processus d'AIP (arc ion plating) et/ou un processus de MSIP (magnetron sputter ion plating) et/ou un processus hybride d'AIP+MSIP.

**9.** Méthode selon la revendication 8, **caractérisé par le fait que** pendant le processus de revêtement au moins une cible solide contenant un ou plusieurs biocides est appliquée comme source de biocide.

**10.** Méthode selon la revendication 9, **caractérisé par le fait que** la méthode comprend une étape dans laquelle au moins une cible contenant des biocides est manoeuvrée au moyen de techniques MSIP et, simultanément, au moins une cible ne contenant pas de biocides est manoeuvrée au moyen de techniques AIP dans une atmosphère de gaz noble / de gaz réactif, la concentration de biocides dans les couches citées comprenant des biocides étant réglée en variant la puissance de pulvérisation de la au moins une cible contenant des biocides et/ou en variant le courant d'arc de la au moins une cible ne contenant pas de biocides.

**11.** Méthode selon l'une des revendications précédentes 7 à 10, **caracterisée par le fait que** la surface du corps de base (2) est soumise à un traitement mécanique ou électrochimique avant le revêtement.

**12.** Méthode selon l'une des revendications précédentes 7 à 11, **caracterisée par le fait que** la surface revêtue (10) est soumise à un traitement mécanique, en particulier à un traitement polissant, aprè le revêtement.

**Haftfestigkeit vs. Silberkonzentration
von Ag/TiN-Schichtsystemen auf Leg. Kaltarbeitsstahl 1.2842**

Fig. 1a

HF1   HF2   HF3   HF4   HF5   HF6

Fig.1b

**Härteabnahme bei Zunahme der Silberkonzentration
von Ag/TiN-Schichtsystemen auf Leg. Kaltarbeitsstahl 1.2842**

Fig.2

**Rauheitserhöhung bei Zunahme der Silberkonzentration
von Ag/TiN-Schichtsystemen auf Leg. Kaltarbeitsstahl 1.2842**

Fig.3

## Optische Eigenschaften bei Zunahme der Silberkonzentration von Ag/TiN-Schichtsystemen auf Leg. Kaltarbeitsstahl 1.2842

Fig.4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008001014 A1
- WO 2009095705 A2

- WO 2009095705 A